(19) Europäisches Patentamt
European Patent Office
Office européen des brevets

(11) EP 2 873 422 A1

(12) **EUROPEAN PATENT APPLICATION**
published in accordance with Art. 153(4) EPC

(43) Date of publication:
**20.05.2015 Bulletin 2015/21**

(21) Application number: **13816710.1**

(22) Date of filing: **09.07.2013**

(51) Int Cl.:
***A61K 38/00*** *(2006.01)*
***A61K 9/08*** *(2006.01)*
***A61K 39/395*** *(2006.01)*
***A61K 47/16*** *(2006.01)*
***A61P 43/00*** *(2006.01)*

(86) International application number:
**PCT/JP2013/068735**

(87) International publication number:
**WO 2014/010586 (16.01.2014 Gazette 2014/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.07.2012 JP 2012154905**

(71) Applicant: **Takeda Pharmaceutical Company Limited**
**Osaka-shi, Osaka 541-0045 (JP)**

(72) Inventors:
- **KIYOSHIMA Kenichiro**
  **Osaka-shi**
  **Osaka 532-0024 (JP)**
- **HORIUCHI Shohei**
  **Osaka-shi**
  **Osaka 532-0024 (JP)**
- **SATO Tomomi**
  **Osaka-shi**
  **Osaka 532-0024 (JP)**
- **IKEDA Megumi**
  **Yaizu-shi**
  **Shizuoka 425-0072 (JP)**

(74) Representative: **Weickmann & Weickmann**
**Postfach 860 820**
**81635 München (DE)**

# (54) PHARMACEUTICAL PREPARATION FOR INJECTION

(57) The present invention provides a preparation for injection comprising a protein with a molecular weight of 20 kDa or greater and a pyridine derivative, wherein the preparation for injection does not comprise a hydrophobic preservative.

EP 2 873 422 A1

## Description

### Technical Field

The present invention relates to a preparation for injection comprising a protein with a molecular weight of 20 kDa or greater and a pyridine derivative, wherein the preparation for injection does not comprise a hydrophobic preservative.

[Background of the Invention]

Proteins with physiological activity are known that exhibit various manners of pharmacological action in the body, and efforts are being made toward their application as drugs. However, since proteins generally tend to decompose in the gastrointestinal tract and digestive organs, they are not administered orally but rather administered by injection. When proteins are stored for prolonged periods, problems occur such as agglutination, denaturation, decomposition, generation of insoluble contaminants or reduction in activity, and it has been difficult to select the solvents and additives for use in formulations. Furthermore, in consideration of convenience for patients, it is preferred to develop subcutaneous injections suitable for self-administration. With proteins, however, agglutination, denaturation, decomposition, generation of insoluble contaminants and reduction in activity occur in the subcutaneous tissue, often making it impossible to achieve the desired absorption rate.
Much research has been conducted on formulation of proteins, with the purpose of dealing with the particular problems of proteins. For example, research has been conducted on stabilization by addition of sucrose (Journal of Pharmaceutical Sciences Vol. 88, No. 3, 1999, J. Peptide Res. 66, 2005 348-356, Journal of Pharmaceutical Sciences, Vol. 98, No. 9, 2009), and increasing the absorption rate of proteins by addition of nicotinic acid amide and improving absorption using hyaluronidase (Diabetes Technology & Therapeutics, Volume 11, Number 6, 2009).

Patent Literature 1 discloses insulin preparations containing nicotinic acid or nicotinamide, and teaches that the absorption rate of insulin (approximately 6 kDa) was increased in pigs.

Patent Literature 2 discloses a pharmaceutical composition comprising a peptide poorly soluble in aqueous physiological saline solution and a non-ionic aromatic hydrotropic pharmaceutically acceptable agent such as nicotinic acid amide or nicotinic acid. Patent Literature 2 teaches that when nicotinic acid amide is added to an aqueous peptide drug solution that normally has low solubility, the solubility of the peptide increases *in vitro* and stability is increased. As examples of peptides, Patent Literature 2 mentions growth hormone releasing factor (GRF, molecular weight: approximately 5 kDa) peptide and luteinizing hormone-releasing hormone (LHRH) antagonist (10 amino acid residue peptide).

Patent Literature 3 discloses a soluble formulation comprising a medically useful peptide or protein, a hydrophobic preservative, and nicotinamide. The soluble formulation has a hydrophobic preservative such as cresol as an essential component, and the physical stability of the medically useful peptide or protein is increased in the presence of the hydrophobic preservative. As peptides or proteins there are mentioned GLP-1, insulin, growth hormones and growth factors.

In Patent Literature 4 and Patent Literature 5 there are disclosed a monoclonal antibody that binds and neutralizes human hepatocyte growth factor (HGF).

Patent Literature 6 discloses an isolated antibody which specifically binds human CD38 and cynomolgus monkey CD38.

However, these prior art documents make no mention of increasing protein absorption *in vivo* by addition of nicotinic acid amide, nicotinic acid or the like.

On the other hand, the absorption property of proteins in the body is problematic when administering protein preparations subcutaneously, intradermally or intramuscularly, for example. Hyaluronidase is among the additives that have been used as absorption aids in the prior art. However, when a protein preparation comprising hyaluronidase is administered subcutaneously, this has caused temporary destruction of the subcutaneous tissue at the site of hyaluronidase administration, or decomposition of the protein by hyaluronidase. Depending on the type of protein, the protein absorption property sometimes fails to be improved even with addition of hyaluronidase.

### Citation List

### Patent Literature

[Patent Literature 1] WO91/009617
[Patent Literature 2] WO98/053844

[Patent Literature 3] WO00/015224
[Patent Literature 4] WO2005/107800
[Patent Literature 5] WO2007/115049
[Patent Literature 6] WO2012/092612

## Summary of Invention

### Technical Problem

The present invention has been accomplished in light of these circumstances, and its object is to provide a preparation for injection that allows administration of proteins with high bioavailability *in vivo,* compared to conventional pharmaceutical protein preparations.

### Solution to Problem

As a result of much diligent research directed toward solving the problems described above, the present inventors have completed this invention upon finding that by administering a combination of the desired protein and a pyridine derivative, it is possible to increase absorption of the desired protein and increase its bioavailability.
Specifically, the present invention provides the following [1] to [6].

[1] A preparation for injection comprising a protein with a molecular weight of 20 kDa or greater and a pyridine derivative, wherein the preparation for injection does not comprise a hydrophobic preservative.
[2] A preparation for injection comprising a protein with a molecular weight of 20 kDa or greater and a pyridine derivative, having improved bioavailability of the protein with a molecular weight of 20 kDa or greater.
[3] The preparation for injection according to [1] or [2], wherein the pyridine derivative is niacin.
[4] The preparation for injection according to [1] or [2], wherein the pyridine derivative is nicotinic acid amide.
[5] The preparation for injection according to [1] or [2], wherein the protein with a molecular weight of 20 kDa or greater is an antibody.
[6] The preparation for injection according to [1] or [2], which is for intradermal administration, subcutaneous administration or intramuscular administration.

The invention further provides the following [7] to [13].

[7] A method for improving bioavailability of a protein with a molecular weight of 20 kDa or greater, comprising administering an effective dose of the preparation for injection according to any one of [1] to [6] to a mammal.
[8] The method according to [7], wherein the mammal is a human.
[9] A pyridine derivative for using in a method for improving bioavailability of a preparation for injection comprising a protein with a molecular weight of 20 kDa or greater.
[10] The pyridine derivative according to [9], wherein the pyridine derivative is niacin.
[11] The pyridine derivative according to [9], wherein the pyridine derivative is nicotinic acid amide.
[12] The pyridine derivative according to [9], wherein the protein with a molecular weight of 20 kDa or greater is an antibody.
[13] The pyridine derivative according to [9], wherein the preparation for injection is for intradermal administration, subcutaneous administration or intramuscular administration.

### Advantageous Effects of Invention

According to the invention it is possible to provide a preparation for injection that allows administration of proteins with high bioavailability *in vivo,* compared to conventional pharmaceutical protein preparations. The preparation for injection of the invention has excellent safety, as it allows administration of a protein into the biological body without destruction of subcutaneous tissue.
Moreover, according to the invention, production efficiency is high since an injection preparation can be provided with low doses compared to conventional protein preparations.
In addition, even when the desired protein is a protein with low solubility, using the invention can provide a low-dose injection preparation allowing the desired protein to be dissolved. Consequently, the present invention has excellent convenience since it allows intradermal administration or subcutaneous administration of proteins with low solubility, that have been difficult to prepare as injections in the prior art.

[Detailed Description of the Invention]

Preferred embodiments of the invention will now be described in detail. However, the present invention is not limited to the embodiments described below.

A preparation for injection according to an embodiment of the invention comprises a protein and a pyridine derivative, and does not comprise a hydrophobic preservative. The preparation for injection having such a construction can increase absorption of the protein and improve bioavailability.

The preparation for injection of this embodiment comprises at least one type of protein as an active ingredient. The protein for this embodiment may be any of various proteins having physiological activity useful for mammals, and being clinically useful for prevention or treatment of any of various diseases in mammals. The protein of this embodiment is not particularly restricted so long as it is a protein having two or more amino acids that are amide bonded, and having physiological activity by formation of a higher-order structure. Thus, compounds having small numbers of amino acids, commonly referred to as peptides or polypeptides, are also included in the concept of protein according to the invention. For example, the protein may be a protein with a molecular weight of about 20 kDa to 1000 kDa, preferably a protein with a molecular weight of about 20 kDa to 500 kDa, and even more preferably a protein with a molecular weight of about 20 kDa to 160 kDa, as a monomer.

Representative proteins with physiological activity include hormones and antibodies. The action mechanisms of the proteins may be either agonistic or antagonistic. Specific examples of proteins for this embodiment include peptide hormones, cytokines, peptidergic neurotransmitters, hematopoietic factors, various growth factors, enzymes, peptide antibiotics, analgesic peptides and antibodies.

Examples of peptide hormones include insulin, somatostatin, somatostatin derivatives (sandostatin, see US 4,087,390, US 4,093,574, US 4,100,117 and US 4,253,998), growth hormone (GH), natriuretic peptide, gastrin, prolactin, adrenocorticotropic hormone (ACTH), ACTH derivatives (ebiratide and the like), melanocyte-stimulating hormone (MSH), thyrotropin-releasing hormone (TRH), as well as its salts and derivatives (see JP S50-121273 and JP S52-116465), thyroid-stimulating hormone (TSH), luteinizing hormone (LH), follicle-stimulating hormone (FSH), human chorionic gonadotropin (HCG), thymosin (thymosine), motilin, vasopressin, vasopressin derivatives [desmopressin see Journal of the Japan Endocrine Society, Vol.54, No. 5, p.676-691(1978)], oxytocin, calcitonin, parathyroid hormone (PTH), glucagon, secretin, pancreozymin, cholecystokinin, angiotensin, human placental lactogen, glucagon-like peptide (GLP-1) and its derivatives (see JP H06-80584, JP H07-2695, EP658568, JP H08-245696, JP H08269097, WO97/15296, WO97/31943, WO98/19698, WO98/43658, JP H10-511365, WO99/55310, JP H11-513983, CA2270320, WO99/64061, JP H11-514972, JP 2000-500505, WO2000/66138, WO2000/66142, WO2000/78333, JP 2001-11095, Tissue Eng. 7(1) 35-44(2001), Diabetologia 43(10)1319-1328(2000), WO2000/34331, WO2000/34332, US 6,268,343, US 2001011071, US 2001006943, EP0733644, WO2000/77039 WO99/43707, WO99/43341, WO99/43706, WO99/43708, WO99/43705, WO99/29336, WO2000/37098, EP0969016, US 5,981,488, US 5,958,909, WO93/25579, WO98/43658, EP0869135, US 5,614,492, US 5,545,618, US 5,120,712, US 5,118,666, WO95/05848, WO91/11457, EP0708179, WO96/06628, EP0658568 and WO87/06941), and metastatin and its derivatives (see WO2000/24890). Preferable peptide hormones include insulin and growth hormones.

Examples of cytokines include lymphokines and monokines. Examples of lymphokines include interferons (alpha-form, beta-form, gamma-form, etc.) and interleukins (for example, IL-2, 3, 4, 5, 6, 7, 8, 9, 10, 11 and 12). Examples of monokines include interleukin-1 (IL-1) and tumor necrosis factor (TNF). Cytokines are preferably lymphokines, more preferably interferon, and particularly preferably interferon alpha.

Examples of peptidergic neurotransmitters include substance P, serotonin and GABA.

Examples of hematopoietic factors include erythropoietin (EPO), colony-stimulating factors (e.g. G-CSF, GM-CSF, M-CSF), thrombopoietin (TPO), platelet growth stimulating factors, megakaryocyte potentiators, and the like.

Examples of various growth factors include basic and acidic fibroblast growth factors (FGF) and their families (for example, EGF, TGF-$\alpha$, TGF-$\beta$, PDGF, acidic FGF, basic FGF, FGF-9 and the like), nerve growth factors (NGF) and their families (for example, BDNF, NT-3, NT-4, CNTF, GDNF and the like), insulin-like growth factors (for example, IGF-1, IGF-2 and the like), and bone growth-related factors (BMP) and their families.

Examples of enzymes include superoxide dismutase (SOD), urokinase, tissue plasminogen activator (tPA), asparaginase and kallikrein. Examples of peptide antibiotics include polymyxin B, colistin, gramicidin and bacitracin.

Examples of analgesic peptides include enkephalins, enkephalin derivatives (see US 4,277,394 and EP-A031567), endorphins and kyotorphin.

Other physiologically active peptides include thymopoietin, dynorphin, bombesin, cerulein, thymostimulin, thymus humoral factor (THF), serum thymic factor (FTS) and its derivatives (see US Patent No. 4,229,438), other thymic factors [Igaku no Ayumi, Vol.125, No. 10, p.835-843(1983)], and neurotensin, bradykinin and peptides with endoserine antagonism (see EP-A436189, EP-A457195, EP-A496452, Japanese Unexamined Patent Application Publication HEI No. 3-94692 and Japanese Unexamined Patent Application Publication HEI No. 3-130299).

A preferred physiologically active peptide to be used in the preparation for injection of this embodiment is peginterferon

alpha-2b (gene recombinant) (PEG-IFNα-2b).
Another physiologically active peptide preferred for use in the preparation for injection of this embodiment is erythropoietin (EPO). Another physiologically active peptide preferred for use in the preparation for injection of this embodiment is vascular endothelial growth factor (VEGF).

The protein with physiological activity for this embodiment may be natural, synthetic or semisynthetic, and so long as it has that physiological activity it may be a derivative, analog or modified form thereof.
The protein with physiological activity for this embodiment may also be in the form of a salt. Salts of proteins with physiological activity for this embodiment may be salts with acids or bases, which are physiologically acceptable salts, and especially preferred are physiologically acceptable acid addition salts. Examples of such salts include salts of inorganic acids (for example, hydrochloric acid, phosphoric acid, hydrobromic acid and sulfuric acid), and salts of organic acids (for example, acetic acid, formic acid, propionic acid, fumaric acid, maleic acid, succinic acid, tartaric acid, citric acid, malic acid, oxalic acid, methanesulfonic acid and benzenesulfonic acid).
The C-terminus of the protein used for this embodiment may be a carboxyl group (-COOH), carboxylate ($-COO^-$), amide ($-CONH_2$) or ester (-COOR).
Examples for R in an ester include $C_{1-6}$ alkyl groups such as methyl, ethyl, n-propyl, isopropyl and n-butyl, $C_{3-8}$ cycloalkyl groups such as cyclopentyl and cyclohexyl, $C_{6-12}$ aryl groups such as phenyl and α-naphthyl, phenyl-$C_{1-2}$ alkyl groups such as benzyl and phenethyl, $C_{7-14}$ aralkyl groups including α-naphthyl-$C_{1-2}$ alkyl groups such as α-naphthylmethyl, and pivaloyloxymethyl.
When the protein used for this embodiment has a carboxyl group (or carboxylate group) other than at the C-terminus, a protein having amidated or esterified carboxyl groups other than on the C-terminus is also included within proteins used for the invention. Examples of esters in this case include the aforementioned esters used for the C-terminus.
In addition, the protein to be used for this embodiment includes proteins wherein the amino group of the N-terminal amino acid residue (for example, a methionine residue) is protected with a protecting group (for example, a $C_{1-6}$ acyl group including a $C_{1-6}$ alkanoyl, such as formyl or acetyl), proteins wherein an N-terminal glutamine residue produced by cleavage in the biological body is pyroglutamated, proteins wherein a substituent on a side chain of an amino acid in the molecule (for example, -OH, -SH, amino group, imidazole group, indole, guanidino group or the like) is protected with a suitable protecting group (for example, a $C_{1-6}$ acyl group including a $C_{1-6}$ alkanoyl, such as formyl or acetyl), and conjugated proteins such as glycoproteins with bonded sugar chains.
The protein of this embodiment may be a protein modified with a molecule having protein stabilizing action, such as polyethylene glycol. Proteins according to this embodiment also include partial peptides of proteins. Partial peptides of proteins to be used for this embodiment may be peptides having partial amino acid sequences of proteins to be used for this embodiment, and they may be any peptides so long as they have essentially the same activity as those proteins. Here, "essentially the same activity" has the same meaning as above. That is, "essentially the same activity" means activity of the same quality (for example, physiochemical or pharmacological) as the aforementioned "physiological activity". Measurement of "essentially the same activity" can be accomplished in the same manner as for a protein to be used for this embodiment as described above.

Preferred examples of proteins for this embodiment include antibodies.

An antibody for this embodiment may be an antibody for any portion of an antigen as the antigenic determinant, so long as it can recognize the antigen in its native structure. The antibody is preferably an antibody that recognizes the extracellular domain of a membrane protein. An antibody for this embodiment that recognizes an extracellular domain can bind antigen expressed on the surfaces of intact cells, and is therefore particularly advantageous for use as an antibody drug.

Antibodies for this embodiment include publicly known isotypes and subtypes. For human-derived antibodies, examples of isotypes include IgM, IgG, IgA, IgD and IgE. The IgG subtype includes, for example, IgG1, IgG2, IgG3 and IgG4. For human-derived antibodies, the light chain includes, for example, the kappa chain and lambda chain. The preferred isotype is IgG.

An antibody may be either a monoclonal antibody or a polyclonal antibody. The antibody may also be an antibody that has been modified by genetic engineering, such as a chimeric antibody and humanized antibody.
The antibody for this embodiment is not particularly restricted so long as it is a molecule having at least a complementarity determining region (CDR) for specifically recognizing and binding a target antigen, and it may be one or more types of molecules selected from the group consisting of complete antibody molecules, fragments such as Fab, Fab', $F(ab')_2$, Fv or VH, conjugate molecules prepared by genetic engineering such as scFv, scFv-Fc, dsFv, a minibody or a diabody, derivatives thereof modified with molecules having protein stabilizing action, such as polyethylene glycol (PEG), and multispecific antibodies.

The method of producing the antibody is not particularly restricted, and any method of producing antibodies that is known in the relevant field may be employed. Examples of methods for producing antibodies include hybridoma methods, methods using microorganisms containing antibody genes introduced by genetic engineering, and methods of directly obtaining antibodies from serum of immunized animals.

The preparation for injection of this embodiment may employ an antibody for any antigen so long as it is an antibody commonly used for treatment. As a preferred antibody to be used in the preparation for injection of this embodiment there may be mentioned anti-hepatocyte growth factor antibody (hereunder also referred to as "anti-HGF antibody").

When a monoclonal antibody that binds with HGF (i.e., "anti-HGFmAb") partially or completely inhibits one or more modes of bioactivity of HGF by binding with HGF, it is said that HGF is neutralized or has been neutralized. Such antibodies are known as neutralizing antibodies. Examples of biological properties of HGF that can be inhibited by neutralizing antibodies include binding to cMet receptor; eliciting scattering of specific cell lines such as Madin-Darby canine kidney (MDCK) cells; stimulating proliferation of specific cells such as hepatocytes, MvlLu mink lung epithelial cells or various human tumor cells; and stimulating vascularization, such as measured, for example, by proliferation of Human Umbilical Vein Endothelial Cells (HUVEC) or angiogenesis, or by inducing blood vessels upon application of Chick Chorioallantoic Membrane (CAM). Humanized neutralizing mAb preferably binds with human HGF (i.e. the protein encode by a polynucleotide having the sequence of GenBank Accession No. D90334). A humanized antibody is a monoclonal gene recombinant antibody having the CDR from a mouse antibody ("donor antibody", which may also be an antibody from rat, hamster or another similar species), transferred onto a human antibody ("receptor antibody", also sometimes referred to as "receptor").

For example, a solution containing humanized neutralizing mAb in a concentration of 0.01, 0.1, 0.5, 1, 2, 5, 10, 20 or 50 μg/ml inhibits a biological function of HGF (for example, stimulating proliferation or scatter) to a degree of at least about 50%, preferably 75% or greater, more preferably 90% or greater, even more preferably 95% or greater, yet more preferably 99% or greater and most preferably about 100% (essentially completely). When the molar ratio of antibody to HGF is 0.1-fold, 0.5-fold, 1-fold, 2-fold, 3-fold, 5-fold or 10-fold, preferably at least 25%, 50%, 75%, 90%, 95% or essentially complete inhibition is attained. More preferably, mAb neutralizes not just one but several of the aforementioned bioactivities of HGF. An anti-HGFmAb that neutralizes all of the bioactivity of HGF is referred to as "completely neutralizing", and such an mAb is most preferred. The humanized neutralizing mAb preferably binds specifically with HGF. The humanized neutralizing mAb preferably does not bind, or binds only to a very low degree, with proteins related to HGF, such as fibroblast growth factor (FGF) and vascular endothelial growth factor (VEGF).

The humanized neutralizing mAb includes anti-HGF antibody in the tetrameric form itself (the form of the naturally occurring tetramer with two L chains and two H chains). The humanized neutralizing mAb may be any of the known isotypes IgG, IgA, IgM, IgD and IgE, as well as other subtypes such as IgG1, IgG2, IgG3 and IgG4. The humanized neutralizing mAb may also contain a κ L chain or a λ L chain. Examples for the humanized neutralizing mAb include antibody fragments such as Fv, Fab and $F(ab')_2$; bifunctional conjugated antibodies; single-stranded antibodies; and antibodies with modified constant regions. The source of the CDR for the humanized neutralizing mAb may be an animal (for example, a mouse, rat, hamster and chicken). The animal-derived CDR may be incorporated into the humanized neutralizing mAb by gene recombination. Rodent mAb is produced by a standard method known in the technical field, such as the one described below, for example. First, HGF in an appropriate adjuvant is used for multiple intraperitoneal, intravenous or intraplantary immunizations in a rodent. Next, spleen or lymph node cells are isolated from the immunized rodent and are fused with an appropriate immortalized cell line to produce a hybridoma. Hybridomas producing HGF-binding antibody are then selected.

Humanized forms of L2G7mAb (humanized antibodies) include most or all of the CDR amino acids of the L2G7mAb H chain and L chain sequences in a human variable region framework (including single, synthetic or consensus sequence human frameworks). For example, there exist humanized antibodies containing three intact CDRs from the H chain of L2G7mAb and three intact CDRs from the L chain of L2G7mAb, and humanized antibodies containing at least one intact CDR from the H chain of L2G7mAb and at least one intact CDR from the L chain of L2G7mAb. Some humanized antibodies comprise at least one CDR wherein several residues are derived from the corresponding CDR of L2G7mAb and the other residues are derived from the CDR of a human antibody, preferably the same human antibody that supplies the variable region framework that includes the CDR.

In some humanized antibodies, at least 1, 3, 5 or all of the positions selected from the group consisting of H29, H30, H48, H66, H67, H71, H94, L3 and L60, are occupied by amino acids correspondingly present in mouse L2G7 monoclonal antibody, based on Kabat numbering. In the variable region framework for the human receptors described below (AUC18323 and BAC01726), these positions are all occupied by human-derived amino acid residues that differ from the amino acid residues present at the symmetrical positions in mouse L2G7 monoclonal antibody. Thus, most or all of the positions selected from the aforementioned group are preferably substituted by human-derived amino acid residues. When another human variable region framework is to be used, those positions can be occupied by the same amino acids as in the human variable region framework and mouse L2G7 monoclonal antibody. Thus, substitution is not carried out at those positions, but rather at other positions that differ between the human variable region framework and the mouse L2G7 monoclonal antibody, according to the rules of Queen. Other amino acids, that are unrelated to the human variable region framework, may also be substituted in addition to the amino acids specified by the aforementioned group. Generally, however, both the H chain variable region framework and L chain variable region framework

of a humanized antibody do not include more than 10 or 12 substitutions that produce residues not present in the receptor human variable region framework (including the human consensus variable region framework and synthetic human variable region framework).

Any constant region present in the humanized antibody is human or essentially equivalent to human, and has less than 10 and preferably no greater than 2 substitutions relating to the natural human constant region. Some substitutions are advantageous for increasing the half-life of the antibody and affinity for FcγRn. Other substitutions and common conservative substitutions are not problematic in practice.

One example of a humanized form of L2G7 (hereunder also referred to as "humanized L2G7") includes the mature H chain variable region and mature L chain variable region shown in Fig. 2 of WO2007/115049. Other preferred forms of humanized L2G7 include the mature H chain variable region and mature L chain variable region having amino acid sequences with at least 90%, 95%, 98% or 99% sequence identity with respect to these amino acid sequences (aligned according to Kabat numbering), and/or include the mature H chain variable region and mature L chain variable region having amino acid sequences that differ from these sequences by a few substitutions, deletions and/or insertions (typically including less than 5 or 10 amino acids) that are not functionally essential. For example, the first amino acid of the H chain may be either Glu or Gin. The substitution is usually conservative. In other words, one amino acid is substituted with a chemically similar amino acid. Amino acids are grouped in the following manner for purposes of classifying amino acid substitutions as conservative or non-conservative: Group I (hydrophobic side-chain): Met, Ala, Val, Leu, Ile; Group II (neutral hydrophilic side chain): Cys, Ser, Thr; Group III (acidic side chain): Asp, Glu; Group IV (basic side chain): Asn, Gln, His, Lys, Arg; Group V (residues that affect chain orientation): Gly, Pro; and Group VI (aromatic side chain): Trp, Tyr, Phe. A conservative substitution is a substitution between amino acids in the same group. Substitutions relating to the variable region of mouse L2G7 monoclonal antibody preferably avoid the H29, H30, H48, H66, H67, H71, H94, L3 and L60 positions. These positions have interaction with the CDR, and include amino acids from mouse L2G7 monoclonal antibody. The substitutions are preferably at the variable region framework positions, but may also occur in the CDR region. For a substitution in the CDR region, preferably an amino acid from the mouse L2G7 monoclonal antibody is preferably substituted with an amino acid at the corresponding position (Kabat numbering) of a human antibody, and preferably the same human antibody that provides the receptor variable region framework.

Usually, humanized L2G7mAb is the IgG1, IgG2, IgG3 or IgG4 isotype having the κ L chain. Throughout the present specification, IgG1mAb having the variable region described in Fig. 2 of WO2007/115049, combined with the complete human γ-1 and κ constant regions, will be referred to as "HuL2G7".

Mutant HuL2G7 retaining an antigen-binding property similar to HuL2G7 can be obtained by mass selection using the phage display method, following mutagenesis. The mutants are first selected with regard to specific binding with HGF, optionally in competition with HuL2G7 or mouse L2G7 monoclonal antibody (hereunder also referred to as "mouse L2G7" and "L2G7"). Next, the mutants having binding properties similar or identical to HuL2G7 or mouse L2G7 antibody may be tested in terms of function.

A preferred humanized L2G7mAb is neutralizing or completely neutralizing on HGF, as defined above. Preferably, the neutralizing activity of the humanized mAb for some, most or all of the measured biological properties of HGF (for example, binding with cMet receptor, or stimulating proliferation of Mv1Lu mink lung epithelial cells or HUVEC cells), is within 3 times the amount (at least 1/3 activity) and more preferably within 2 times the amount (at least 1/2 activity) or 1.5 times the amount (at least 2/3 activity) for the neutralizing activity of L2G7 itself, and most preferably it is approximately equivalent to the neutralizing activity of L2G7 itself (within experimental error). In other words, at most 3 times, 2 times, 1.5 times or the same amount of humanized mAb with respect to L2G7 is necessary to obtain the same level of inhibition of the biological properties (as measured by IC50, for example). The affinity of the humanized mAb for HGF is preferably within 3 times the amount (at least 1/3 affinity for HGF), within 2 times the amount (at least 1/2) or approximately equivalent to that of L2G7. Similarly, in a mouse heterograft model (for example, mice in which a human glioma cell line such as U87 is used as the heterograft), the humanized mAb inhibits tumor growth with an amount of preferably within 3 times or within 2 times the amount of mouse L2G7mAb, or in an amount equivalent to mouse L2G7mAb. In actuality, growth of U87 tumor heterograft is preferably completely inhibited by twice weekly administration of the humanized mAb at a dose of 40, 20 or 10 μg.

The humanized mAb can be expressed by any of various methods known to those skilled in the art. For example, genes coding for the L chain and H chain variable regions of L2G7 are synthesized by PCR mutagenesis using a redundant oligonucleotide or a previously prepared mutant of the target gene as template. Because of degeneracy of the genetic code, several DNA sequences code for each antibody amino acid sequence. However they are prepared, the genes coding for the humanized mAb L chain and H chain are inserted into an expression vector providing the necessary control regions such as the promoter, enhancer and polyA site (for example, a commercially available expression vector by Invitrogen Corp.), together with the constant region. The use of CMV promoter-enhancer is preferred. Genes for constant regions are currently in wide use, and can be easily cloned from human antibody-producing cells using PCR methods. The L chain gene and H chain gene may be inserted into a single vector or into separate vectors. The expression vector may then be transfected into any of various mammalian cell lines such as a non-producing

myeloma including CHO cells or 293 cells (HEK293 cells), Sp2/0 cells and NS0 cells, by a method known to those skilled in the art such as lipofection or electroporation. Next, antibody-producing cells are selected using an appropriate antibiotic. Greater amounts of antibody can be produced by growing the cells in a commercially viable bioreactor.

Once expressed, the humanized mAb may be purified by standard means known to those skilled in the art, such as microfiltration, ultrafiltration, Protein A or Protein G affinity chromatography, size exclusion chromatography, anion exchange chromatography, cation exchange chromatography, or other forms of affinity chromatography based on organic dyes or the like. For pharmaceutical use, an essentially pure antibody with at least about 90 or 95% homogeneity is preferred, with at least 98% or 99% homogeneity being most preferred.

There are no particular restrictions on the method for producing monoclonal antibodies for HGF, and they may be produced by the following procedure, for example.

The first step toward humanized L2G7 is cloning of the L2G7 L chain gene and H chain gene. RNA is prepared from $10^6$ cells of L2G7 (IgG2a, κ) hybridoma cells using an RNeasy Mini Kit (Qiagen Inc.). Next, using a Stratagene kit, single-stranded cDNA is synthesized from the obtained RNA using a random primer, and this is appended to single-stranded cDNA having a dG tail synthesized by terminal deoxynucleotide transferase (Promega Corp.). Using a high performance polymerase AccuPrime Pfx (Invitrogen Corp.), the H chain variable region gene and L chain variable region gene are each amplified from the cDNA using a primer that anneals to the dG tail, a primer that anneals to the N-terminal region of Cγ2a for the H chain and a primer that anneals to the N-terminal region of Cκ for the L chain. Bands of appropriate size are gel-purified from the PCR product, and the dideoxy termination method is used for direct sequencing or sequencing after cloning, using an automatic sequencer.

In order to express an L2G7 chimera and subsequently the humanized mAb, expression vectors analogous to pVk and pVg1 vectors (J. Immunol. 148:1149, 1992) containing the human Cκ gene and human Cγ1 gene, respectively, are constructed from commercially viable vectors and DNA fragments. The L chain vector has a hyg-selectable marker instead of gpt, while the H chain vector has a neoselectable marker instead of Dhfr. The cloned VL gene and VH gene are each subcloned in the appropriate sites of their respective vectors, to produce an L chain gene expression plasmid and H chain gene expression plasmid for chimeric L2G7 (chL2G7) mAb. It is confirmed that chL2G7mAb is produced and that it binds with HGF to approximately the same degree as L2G7, and therefore that the proper L chain and H chain variable regions have been cloned.

The method of Queen et al. (US5,530,101 and US5,585,089) is generally followed for design of humanized L2G7mAb. The NCBI database of human antibody sequences is scanned, and the human VH sequence AAC18323 and human Vκ sequence BAC01726 are selected. The selected human VH sequence and human Vκ sequence are used as receptor sequences for VH and VL of L2G7, respectively (because the human VH sequence AAC18323 and human Vκ sequence BAC01726 have high framework identity with the VH sequence and VL sequence of L2G7, respectively). The computer program Deep View Swiss-Pdb Viewer, available for use on the internet, is used to construct a molecular model of the L2G7 variable domain. The constructed molecular model is used to determine the positions of amino acids in the L2G7 framework that are sufficiently near for the CDR to interact with the L2G7 framework. In order to design the H chain variable region and L chain variable region for humanized L2G7, the CDR from mouse L2G7mAb is first conceptually transplanted into the receptor framework region. The computer model substitutes amino acids of the original human framework with amino acids from the mouse antibody, at the framework positions that are suggested to be in important contact with the CDR and potentially essential for maintaining the CDR structure. For HuL2G7 (humanized L2G7mAb), the substitution is carried out at residues 29, 30 (within the Chothia hypervariable loop H1), 48, 66, 67, 71 and 94 of the H chain, and residues 3 and 60 of the L chain, according to the Kabat numbering. In addition, the amino acid at position 1 of the H chain is substituted with E (Glu), because its potential for being derivatized is less than that of Q (Gln) in the antibody protein.

HuL2G7, as the representative mAb used in the examples, is IgG1 having the human κ constant region and human γ1 constant region. However, it is to be understood that other IgG1mAb (IgG1, κ) allotypes are included by the reference to HuL2G7. Other isotypes of humanized mAb (for example, IgG2, IgG3 and IgG4) can be prepared by combining the HuL2G7 variable region with appropriate human constant regions. In order to reduce or augment effector functions such as complement-mediated cytotoxicity and antibody-dependent cellular cytotoxicity (ADCC), or to extend the half-life in humans, substitutions may also be made in the HuL2G7 constant region. In particular, HuL2G7 having a mutation to Gln at position 250 and/or a mutation to Leu at position 428 in the IgG constant region is mentioned as an embodiment, but there is no limitation to those.

Once HuL2G7mAb has been designed, i.e. once the amino acid sequences of the L chain variable region and H chain variable region of HuL2G7mAb have been selected, the DNA sequences coding for the variable regions (including the DNA sequences coding for the signal peptides) are selected as usual by the genetic code. These DNA sequences begin from CTCGAGACCACC, as the sequence upstream from the start ATG codon, in order to provide a restriction site for cloning and the Kozak translation initiation signal. These DNA genes can be commercially synthesized by Genscript Corp. (Piscataway, NJ), i.e. synthesis of two pairs of oligonucleotides alternately overlapping on the strands (applying a Biosystems DNA synthesizer). These together include the entire gene. The synthesized oligonucleotides

are oligonucleotides of 110 to 140 nucleotides with overlapping of 15 nucleotides. Double strand DNA fragments are synthesized respectively from the 5' pair and 3' pair of oligonucleotides using Klenow polymerase. The 5'-DNA fragment is cut with a restriction enzyme that cleaves the 5'-end and center of the variable region. The 3'-DNA fragment is cut with a restriction enzyme that cleaves the center and 3'-end of the variable region. Each cut fragment (cut DNA fragment) is inserted into a suitable cloning vector and used to transform *E. coli.* Most of the isolated strain DNA is then sequenced and a DNA fragment is found having the complete proper sequence. Next, each gene is used for ternary ligation, the proper 5' fragment and 3' fragment are inserted into an appropriate expression vector to form the complete gene, and the sequence is confirmed.

For production of HuL2G7mAb, human renal epithelial 293-F cells (Invitrogen Corp.) are cultured in FreeStyle293 expression medium (FS medium; Invitrogen Corp.), and are resuspended in FD medium at $10^6$ cells/2 ml/microwell. The L chain expressing vector DNA and H chain expressing vector DNA (1 μg each) of HuL2G7 are incubated for 30 minutes at room temperature in 100 μl of FS medium together with 3 μl of Fugene 6 (Roche). The incubated mixture is then added to the cells for transfection. After incubation of the cells for 48 hours, the transfected cells are cultured in the presence of 1 mg/ml of G418 and cells expressing the neomycin resistance gene are selected. The selected cells are then developed in a 96-well tissue culture plate (100 μl/well). After approximately 2 weeks, when the wells containing viable cells have reached confluency, the presence and amount of HuL2G7 is tested based on ELISA measurement of the culture supernatants from the wells. The transfected cells may disproportionately secrete the L chain and H chain. Therefore, this ELISA employs goat anti-human Fc antibody as the capture antibody and biotinylated anti-human κ antibody as the detecting reagent, in order to ensure that only the complete HuL2G7 is measured. The chL2G7mAb is expressed similarly. Clones of cells expressing relatively high levels of ChL2G7 and HuL2G7 are respectively expanded and grown in FS medium. The produced antibody is purified from the culture supernatants using protein A affinity chromatography and analyzed for purity by SDS-PAGE.

More specifically, as HuL2G7mAb, a humanized monoclonal antibody of monoclonal antibody L2G7 produced by the hybridoma listed as ATCC No.: PTA-5162, and used in the Example 1 below, is preferably used. Also preferred for use is a humanized monoclonal antibody that competes with the monoclonal antibody produced by the hybridoma listed as ATCC No.: PTA-5162 for binding with HGF. HuL2G7mAb can be produced, for example, according to the method of WO2007/115049.

Another preferred example of an antibody to be used for a preparation for injection according to this embodiment is anti-CD38 antibody. Specific examples for the anti-CD38 antibody include the antibodies mentioned in WO2012/092612, among which Ab79 is preferred.

The preparation for injection of this embodiment may comprise a single type of the aforementioned protein, or a combination of two or more of the proteins.

The preparation for injection of this embodiment may also comprise a pyridine derivative. Pyridine derivatives include hydrophilic pyridine analogs, such as nicotinic acid amide and niacin. Nicotinic acid amide is an amide of niacin (also called nicotinic acid or vitamin B3), and is also known as niacinamide or nicotinamide. Nicotinic acid amide is a water-soluble vitamin belonging to the group B vitamins. Nicotinic acid amide is produced from niacin in the biological body. Structural formula (A) for nicotinic acid amide and structural formula (B) for niacin are shown below.

[Chemical Formula 1]

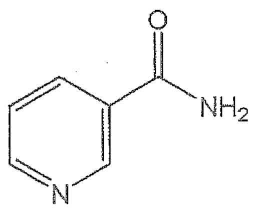

(A)

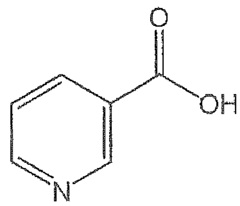

(B)

The pyridine derivative may also be in a salt form. When the pyridine derivative is a salt, examples of such salts include metal salts, ammonium salts, salts with organic bases, salts with inorganic acids, salts with organic acids, salts with basic amino acids and salts with acidic amino acids. Preferred examples of metal salts include alkali metal salts such as sodium salts and potassium salts, alkaline earth metal salts such as calcium salts, magnesium salts and barium salts, and aluminum salts. Preferred examples of salts with organic bases include salts with trimethylamine, triethylamine, pyridine, picoline, 2,6-lutidine, ethanolamine, diethanolamine, triethanolamine, cyclohexylamine, dicyclohexylamine or N,N'-dibenzylethylenediamine. Preferred examples of salts with inorganic acids include salts with hydrochloric acid, hydrobromic acid, nitric acid, sulfuric acid or phosphoric acid. Preferred examples of salts with organic

acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid or p-toluenesulfonic acid. Preferred examples of salts with basic amino acids include salts with arginine, lysine or ornithine. Preferred examples of salts with acidic amino acids include salts with aspartic acid or glutamic acid.

Of these, a pharmaceutically acceptable salt is preferable. For example, when a compound has an acidic functional group, an inorganic salt such as alkali metal salt (e.g., sodium salt, potassium salt etc.), alkaline earth metal salt (e.g., calcium salt, magnesium salt etc.) and the like, ammonium salt etc., and when a compound has a basic functional group, for example, a salt with inorganic acid such as hydrochloric acid, hydrobromic acid, nitric acid, sulfuric acid, phosphoric acid and the like, or a salt with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like can be mentioned.

The pyridine derivative may be produced by a known method, or a commercially available product may be used directly. For example, niacin can be obtained from Yuki Gosei Kogyo Co., Ltd.

The concentration of the pyridine derivative may be set as appropriate, but it is preferably 0.1 to 500 mg/ml, more preferably 1 to 148 mg/ml and even more preferably 10 to 74 mg/ml.

The preparation for injection of this embodiment does not comprise a hydrophobic preservative. Hydrophobic preservatives are hydrophobic compounds that are added to medical formulations and act as antimicrobial agents, and they include, for example, phenolic preservatives such as alkylparabens and cresol, and benzyl alcohol, chlorobutanol and benzoic acid, as well as mixtures thereof. By the phrase "does not comprise a hydrophobic preservative" it is meant either that:

1) the preparation for injection does not contain a hydrophobic preservative, or
2) even if the preparation for injection contains a hydrophobic preservative, its content is "less than the amount in which it acts as an antimicrobial agent".

The "amount in which it acts as an antimicrobial agent" can be determined by referring to Wallhauser, K.H., Develop. Biol. Standard. 24, pp.9-28 (Basel, S. Kranger, 1974), for example.

In other words, "less than the amount in which it acts as an antimicrobial agent" means that the "hydrophobic preservative" is essentially absent (is essentially not comprised) in the injection. Here, the phrase "is essentially absent (is essentially not comprised)" means that it may be present in the injection so long as it is "less than the amount in which it significantly acts as an antimicrobial agent".

Thus, the phrase "does not comprise a hydrophobic preservative" includes both of the following:

1) where absolutely no hydrophobic preservative is present in the preparation for injection of this embodiment; and
2) where the hydrophobic preservative is essentially not present in the preparation for injection of this embodiment.

For this embodiment, the combining weight ratio of the protein and the pyridine derivative is preferably 1:1,000,000 to 10,000:1, more preferably 1:10,000 to 100:1 and even more preferably 1:100 to 30:1.

As a second embodiment of the invention, there may be mentioned a preparation for injection comprising a protein and a pyridine derivative, allowing bioavailability of the protein to be improved. Thus, the invention further provides a method for improving bioavailability of the aforementioned protein, the method comprising administering an effective dose of the preparation for injection to a mammal.

The preparation for injection of this embodiment having the construction described above, when administered into the body, can increase absorption of the protein and improve its bioavailability. For this embodiment, bioavailability of the protein can be determined by measuring to what extent the protein in the preparation for injection is absorbed into the systemically circulating blood when the preparation for injection of this embodiment is subcutaneously, intracutaneously or intramuscularly administered. That is, it can be measured by calculating the percentage of the protein that has been subcutaneously, intracutaneously or intramuscularly administered, that is absorbed into the systemically circulating blood, where direct injection of the preparation for injection into the blood is defined as 100%. The "improved bioavailability" of the protein in the preparation for injection of this embodiment is an increase in bioavailability compared to the protein preparation without addition of the pyridine derivative, and for example, it is protein absorption of greater than 1 to 10,000-fold, preferably 1.001-fold to 1000-fold and more preferably 1.001-fold to 100-fold.

The pH of the preparation for injection according to this embodiment is preferably about 2.5 to 10, more preferably about 3 to 9 and even more preferably about 3 to 7.

The preparation for injection of this embodiment can be produced by mixing the one or more proteins and a pyridine derivative, if necessary also with a pharmaceutically acceptable carrier, by a known method. As pharmaceutically acceptable carriers to be used for production of the preparation for injection of this embodiment there may be mentioned various organic carrier substances or inorganic carrier substances commonly used as formulating materials, examples

of which include solvents, dissolving aids, suspending agents, isotonizing agents, buffering agents, soothing agents and the like. If necessary, common additives such as stabilizers and antioxidants may also be used in suitable amounts as appropriate.

Examples of carriers for the preparation for injection of this embodiment include solvents, dissolving aids, isotonizing agents, buffering agents, stabilizers, soothing agents and the like.

Examples of solvents include water for injection, physiological saline and Ringer's solution, as aqueous solutions. Examples of solvents also include sesame oil and soybean oil, as oily solutions.

Examples of dissolving aids include alcohols (for example, ethanol and the like), polyalcohols (for example, propylene glycol, polyethylene glycol and the like), nonionic surfactants (for example, polysorbate 80™, HCO-50 and the like), D-mannitol, benzyl benzoate, trisaminomethane, cholesterol, triethanolamine, sodium carbonate, sodium citrate, and the like.

Examples of isotonizing agents include glucose, D-sorbitol, sodium chloride, glycerin, D-mannitol and the like.

Examples of buffering agents include phosphate, acetate, carbonate and citrate buffering solutions and the like.

Examples of soothing agents include benzalkonium chloride, procaine hydrochloride and the like.

Examples of stabilizers include human serum albumin, sucrose, trehalose, histidine, arginine, polysorbate, polyethylene glycol and the like.

Examples of antioxidants include sulfurous acid salts, ascorbic acid, $\alpha$-tocopherols and the like.

The preparation for injection of this embodiment may be stored as a powder after lyophilizing in an aseptically treated lyophilizer, or it may be stored in a liquid state, sealed in an injecting container (for example, an ampule). The powder obtained by lyophilizing the preparation for injection of this embodiment can be used by dilution at the time of use with the aforementioned carrier for the preparation for injection.

The preparation for injection of this embodiment can be used as a drug (for example, a prophylactic or treatment agent for a disease) or an animal drug or the like, for a mammal (for example, a rat, mouse, guinea pig, monkey, cow, dog, pig, human or the like).

The preparation for injection of this embodiment can be used for intravenous administration, intramuscular administration, intradermal administration, subcutaneous administration or intraorgan administration, and is preferably used for intramuscular administration, intradermal administration or subcutaneous administration. The preparation for injection may also be directly administered to a focus of disease.

The dose for the preparation for injection of this embodiment will differ depending on the protein type, the patient age, body weight and symptoms, the dosage form, the method of administration, the period of administration and other factors, but for example, it will usually be administered at about 0.0001 to about 1000 mg/kg, preferably about 0.0001 to about 100 mg/kg, more preferably about 0.001 to about 100 mg/kg, even more preferably about 0.01 to about 50 mg/kg and yet more preferably about 0.01 to about 30 mg/kg per day, as the protein of this embodiment, per patient (adult with body weight of approximately 60 kg), either at one time in the day or in divided doses. Naturally, these doses will vary according to various conditions and therefore amounts lower than these doses may be sufficient, while it may be necessary for the administration to exceed these dose ranges.

An "effective dose", for this embodiment, means an effective dose of the protein, and "administer an effective dose" means to administer the preparation for injection of this embodiment containing the effective dose of protein.

## Examples

The invention will now be explained in greater detail through examples. However, the invention is not limited to these examples. In the following examples and comparative examples, the formulation additives used were products conforming to the Japanese Pharmacopoeia, 16th Revision, or Japanese Pharmaceutical Excipients 2003.

### Comparative Example 1

A 60 mL portion of a 10 mg/mL formulation solution of humanized anti-HGF monoclonal antibody HuL2G7mAb mentioned in the examples of WO2007/115049 (hereunder abbreviated as "antibody A") was added to a glass beaker using a pipette (Eppendorf AG). Next, 60 mL of water for injection (product of Otsuka Pharmaceutical Factory, Inc.) was added to the glass beaker with a pipette, to obtain 120 mL of a 5 mg/mL solution of antibody A.

### Example 1

After weighing out 2.93 g of nicotinic acid amide (product of Yuki Gosei Kogyo Co., Ltd.) into a glass vial (product of Daiwa Special Glass Co., Ltd.), a sufficient amount of water for injection (product of Otsuka Pharmaceutical Factory, Inc.) was added with a pipette (product of Eppendorf AG) and the mixture was dissolved. This was then adjusted upward to 20 mL with water for injection to prepare 20 mL of a 1200 mM nicotinic acid amide solution. The 10 mg/mL antibody

A solution and the 1200 mM nicotinic acid amide solution were each transferred in an amount of 2 mL into a glass vial using a pipette and mixed, to prepare 4 mL of a 5 mg/mL antibody A solution containing 600 mM of nicotinic acid amide.

Test Example 1

Sixteen rats (supplied from Clea Japan, Inc., Jcl:SD, specific pathogen-free, 7-week-old, body weight at administration: 240-340 g) were anesthetized with the Japanese Pharmacopoeia isoflurane (product of Mylan Pharmaceutical). The anesthetized rats were divided into two groups, and then a polypropylene glass syringe and a 22 gauge injection needle were used for subcutaneous administration of the solution obtained in Comparative Example 1 to one group and the solution obtained in Example 1 to the other group, through the dorsocervical region, in an administered liquid volume of 0.5 mL/kg each (2.5 mg/kg as the administered dose of antibody A). Care was taken during the administration to pinch the site of entry with fingers during removal of the needle from the site of entry, and immediately after removal a small amount of surgical AronAlpha (product of Daiichi Sankyo Co., Ltd.) was applied to the site of entry to prevent leakage. Each group was further divided into two groups, and blood was collected according to the blood collection schedule shown in the following table. In Table 1, "x" represents the time of blood collection.

[Table 1]

| Animal No. | Sampling point (after administration) | | | | | | | | | |
|---|---|---|---|---|---|---|---|---|---|---|
| End | 5 min | 15 min | 30 min | 3 hr | 6 hr | 1 day | 4 day | 1 wk | 2 wk | 3 wk |
| 01-04<br>Comp. Ex. 1 administered group | x | | x | | x | | x | | x | |
| 05-08<br>Example 1 administered group | x | | x | | x | | x | | x | |
| 09-12<br>Comp. Ex. 1 administered group | | x | | x | | x | | x | | x |
| 13-16<br>Example 1 administered group | | x | | x | | x | | x | | x |

Blood was collected from the jugular vein under the Japanese Pharmacopoeia-listed isoflurane anesthesia, and approximately 0.9 mL was sampled up to the collection point of one day after administration, while approximately 1.5 mL was sampled at other times (collection points of day 4, week 1, week 2 and week 3 after administration). A polypropylene glass syringe and a 25 gauge injection needle were used for blood collection. The collected blood was placed in a glass test tube and stationed at room temperature for approximately 60 minutes (±15 min). Next, the glass test tube was subjected to centrifugal separation (about 1600 × g, 10 minutes, 4°C) and the serum was harvested. The serum was sampled into a polypropylene tube (product of Assist Co., Ltd.) at 300 μL, or more from before administration until 1 day after administration, and at 500 μL or more thereafter. The sampled serum was measured for concentration of antibody A in the serum (antibody A concentration) by ELISA. Plotting antibody A concentration obtained by the measurement on the ordinate and blood collection time on the abscissa, the trapezoidal rule was used to calculate the area under the curve of serum concentration [AUC (ng· day/mL)]. As a result, the AUC was 87347 ng· day/mL in Example 1, and the AUC was 75990 ng· day/mL in Comparative Example 1. Also, the relative bioavailability was calculated by the following formula. Relative bioavailability % in Example 1 = (AUC in Example 1)/(AUC in Comparative Example 1) × 100.
As a result, the relative bioavailability was 114%, confirming an effect of improving subcutaneous absorption of antibody A by addition of nicotinic acid amide.

Comparative Example 2

A 5 mL portion of a 20 mg/mL formulation solution of humanized anti-CD38 monoclonal antibody Ab79 mentioned in the examples of WO2007/115049 (hereunder abbreviated as "antibody B") was added to a glass beaker using a pipette (Eppendorf AG). Next, 15 mL of water for injection (product of Otsuka Pharmaceutical Factory, Inc.) was added to the glass beaker with a pipette, to obtain 20 mL of a 5 mg/mL solution of antibody B.

Example 2

After weighing out 5.86 g of nicotinic acid amide (product of Yuki Gosei Kogyo Co., Ltd.) into a glass vial (product of Daiwa Special Glass Co., Ltd.), a sufficient amount of water for injection (product of Otsuka Pharmaceutical Factory, Inc.) was added with a pipette (product of Eppendorf AG) and the mixture was dissolved. This was then adjusted upward to 40 mL with water for injection to prepare 40 mL of a 1200 mM nicotinic acid amide solution. A 2.5 mL portion of the 20 mg/mL antibody B solution, a 2.5 mL portion of the 1200 mM nicotinic acid amide solution and a 5 mL portion of water for injection (product of Otsuka Pharmaceutical Factory, Inc.) were each transferred into a glass vial using a pipette and mixed, to prepare 10 mL of a 5 mg/mL antibody B solution containing 300 mM of nicotinic acid amide.

Example 3

After weighing out 5.86 g of nicotinic acid amide (product of Yuki Gosei Kogyo Co., Ltd.) into a glass vial (product of Daiwa Special Glass Co., Ltd.), a sufficient amount of water for injection (product of Otsuka Pharmaceutical Factory, Inc.) was added with a pipette (product of Eppendorf AG) and the mixture was dissolved. This was then adjusted upward to 40 mL with water for injection to prepare 40 mL of a 1200 mM nicotinic acid amide solution. A 2.5 mL portion of the 20 mg/mL, antibody B solution, a 5 mL portion of the 1200 mM nicotinic acid amide solution and a 2.5 mL portion of water for injection (product of Otsuka Pharmaceutical Factory, Inc.) were each transferred into a glass vial using a pipette and mixed, to prepare 10 mL of a 5 mg/mL antibody B solution containing 600 mM of nicotinic acid amide.

Test Example 2

Twenty-four rats (supplied from Clea Japan, Inc., Jcl:SD, specific pathogen-free, 7-week-old, body weight at administration: 240-340 g) were anesthetized with the Japanese Pharmacopeia isoflurane (product of Mylan Pharmaceutical). The anesthetized rats were divided into 3 groups, and then a polypropylene glass syringe and a 22 gauge injection needle were used for subcutaneous administration of the solution obtained in Comparative Example 2 to the first group, the solution obtained in Example 2 to the second group and the solution obtained in Example 3 to the third group, through the dorsocervical region, in an administered liquid volume of 1 mL/kg each (5 mg/kg as the administered dose of antibody B). Care was taken during the administration to pinch the site of entry with fingers during removal of the needle from the site of entry, and immediately after removal a small amount of surgical AronAlpha (product of Daiichi Sankyo Co., Ltd.) was applied to the site of entry to prevent leakage. Each group was further divided into two groups, and blood was collected according to the blood collection schedule shown in the following table. In Table 2, X represents the time of blood collection.

[Table 2]

| Animal No. | Sampling point (after administration) | | | | | | | | | |
|---|---|---|---|---|---|---|---|---|---|---|
| End | 5 min | 15 min | 30 min | 3 hr | 6 hr | 1 day | 4 day | 1 wk | 2 wk | 3 wk |
| 17-20<br>Comp. Ex. 2 administered group | x | | x | | x | | x | | x | |
| 21-24<br>Example 2 administered group | x | | x | | x | | x | | x | |
| 25-28<br>Example 3 administered group | x | | x | | x | | x | | x | |
| 29-32<br>Comp. Ex. 2 administered group | | x | | x | | x | | x | | x |
| 33-36<br>Example 2 administered group | | x | | x | | x | | x | | x |
| 37-40<br>Example 3 administered group | | x | | x | | x | | x | | x |

Blood was collected from the jugular vein under the Japanese Pharmacopoeia-listed isoflurane anesthesia, and approximately 0.9 mL was sampled up to the collection point of one day after administration, while approximately 1.5 mL was sampled at other times (collection points of day 4, week 1, week 2 and week 3 after administration). A

polypropylene glass syringe and a 25 gauge injection needle were used for blood collection. The collected blood was placed in a glass test tube and stationed at room temperature for approximately 60 minutes (±15 min). Next, the glass test tube was subjected to centrifugal separation (about 1600 × g, 10 minutes, 4°C) and the serum was harvested. The serum was sampled into a polypropylene tube (product of Assist Co., Ltd.) at 300 μL or more from before administration until 1 day after administration, and at 500 μL or more thereafter. The sampled serum was measured for concentration of antibody B in the serum (antibody B concentration) using Biacore (product of GE Healthcare, appliance name: Biacore T200). Plotting antibody B concentration obtained by the measurement on the ordinate and blood collection time on the abscissa, the trapezoidal rule was used to calculate the area under the curve of serum concentration [AUC (μg·day/mL)]. As a result, the AUC was 879 μg· day/mL in Comparative Example 2, the AUC was 935 μg· day/mL in Example 2 and the AUC was 939 μg· day/mL in Example 3. Also, the relative bioavailability was calculated by the following formula.

$$\text{Relative bioavailability \% in Example 2} = (\text{AUC in Example 2})/(\text{AUC in Comparative Example 2}) \times 100 = 106\%$$

$$\text{Relative bioavailability \% in Example 3} = (\text{AUC in Example 3})/(\text{AUC in Comparative Example 2}) \times 100 = 107\%$$

This confirmed an effect of improving subcutaneous absorption of antibody B by addition of nicotinic acid amide.

## Claims

1. A preparation for injection comprising a protein with a molecular weight of 20 kDa or greater and a pyridine derivative, wherein the preparation for injection does not comprise a hydrophobic preservative.

2. A preparation for injection comprising a protein with a molecular weight of 20 kDa or greater and a pyridine derivative, having improved bioavailability of the protein with a molecular weight of 20 kDa or greater.

3. The preparation for injection according to claim 1 or 2, wherein the pyridine derivative is niacin.

4. The preparation for injection according to claim 1 or 2, wherein the pyridine derivative is nicotinic acid amide.

5. The preparation for injection according to claim 1 or 2, wherein the protein with a molecular weight of 20 kDa or greater is an antibody.

6. The preparation for injection according to claim 1 or 2, which is for intradermal administration, subcutaneous administration or intramuscular administration.

7. A method for improving bioavailability of a protein with a molecular weight of 20 kDa or greater, comprising administering an effective dose of the preparation for injection according to any one of claims 1 to 6 to a mammal.

8. A pyridine derivative for using in a method for improving bioavailability of a preparation for injection comprising a protein with a molecular weight of 20 kDa or greater.

**INTERNATIONAL SEARCH REPORT**

International application No.
PCT/JP2013/068735

A. CLASSIFICATION OF SUBJECT MATTER
*A61K38/00*(2006.01)i, *A61K9/08*(2006.01)i, *A61K39/395*(2006.01)i, *A61K47/16*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K38/00, A61K9/08, A61K39/395, A61K47/16, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | JP 10-502938 A (Red Cross Foundation Central Laboratory Blood Transfusion Service SRC), 17 March 1998 (17.03.1998), claims 1, 3, 4; page 6, lines 18 to 20; examples 5, 6 & WO 96/07429 A1 & US 5871736 A & EP 779818 A1 | 1-6,8 |
| X | R.Bolli et al., 'IgG-dimer formation in liquid immunoglobulin preparations is inhibited by nicotinamide and other amphiphilic compounds.', Journal of Autoimmunity, 1999, Suppl., p.96 | 1-6,8 |
| X | R. Bolli et al., 'L-Proline reduces IgG dimer content and enhances the stability of intravenous immunoglobulin(IVIG) solutions.', Biologicals, 2010, Vol.38, No.1, p.150-157 | 1-6,8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 July, 2013 (24.07.13) | Date of mailing of the international search report<br>06 August, 2013 (06.08.13) |
| Name and mailing address of the ISA/<br>Japanese Patent Office<br><br>Facsimile No. | Authorized officer<br><br>Telephone No. |

Form PCT/ISA/210 (second sheet) (July 2009)

**INTERNATIONAL SEARCH REPORT**

International application No. PCT/JP2013/068735

C (Continuation). DOCUMENTS CONSIDERED TO BE RELEVANT

| Category* | Citation of document, with indication, where appropriate, of the relevant passages | Relevant to claim No. |
|---|---|---|
| X | JP 2002-524514 A (Eli Lilly and Co.), 06 August 2002 (06.08.2002), claim 1; paragraphs [0009], [0018] & WO 00/15224 A1 & US 2003/0069182 A1 & EP 1113779 A1 | 2-4,6,8 |

Form PCT/ISA/210 (continuation of second sheet) (July 2009)

**INTERNATIONAL SEARCH REPORT**

International application No.
PCT/JP2013/068735

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 7
because they relate to subject matter not required to be searched by this Authority, namely:
(See extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2013/068735

Continuation of Box No.II-1 of continuation of first sheet(2)

When "a method comprising - - - administering an effective dose of a preparation for injection" appearing in claim 7 is taken into consideration, it appears that claim 7 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 91009617 A
- WO 98053844 A
- WO 00015224 A
- WO 2005107800 A
- WO 2007115049 A
- WO 2012092612 A
- US 4087390 A
- US 4093574 A
- US 4100117 A
- US 4253998 A
- JP S50121273 A
- JP S52116465 A
- JP H0680584 B
- JP H072695 B
- EP 658568 A
- JP H08245696 B
- JP H08269097 B
- WO 9715296 A
- WO 9731943 A
- WO 9819698 A
- WO 9843658 A
- JP H10511365 B
- WO 9955310 A
- JP H11513983 B
- CA 2270320
- WO 9964061 A
- JP H11514972 B
- JP 2000500505 A
- WO 200066138 A
- WO 200066142 A
- WO 200078333 A
- JP 2001011095 A
- WO 200034331 A
- WO 200034332 A
- US 6268343 B
- US 2001011071 A
- US 2001006943 A
- EP 0733644 A
- WO 200077039 A
- WO 9943707 A
- WO 9943341 A
- WO 9943706 A
- WO 9943708 A
- WO 9943705 A
- WO 9929336 A
- WO 200037098 A
- EP 0969016 A
- US 5981488 A
- US 5958909 A
- WO 9325579 A
- EP 0869135 A
- US 5614492 A
- US 5545618 A
- US 5120712 A
- US 5118666 A
- WO 9505848 A
- WO 9111457 A
- EP 0708179 A
- WO 9606628 A
- EP 0658568 A
- WO 8706941 A
- WO 200024890 A
- US 4277394 A
- EP 031567 A
- US 4229438 A
- EP 436189 A
- EP 457195 A
- EP 496452 A
- JP 3094692 A
- JP 3130299 A
- US 5530101 A, Queen
- US 5585089 A


### Non-patent literature cited in the description


- *Journal of Pharmaceutical Sciences,* 1999, vol. 88 (3
- *J. Peptide Res.,* 2005, vol. 66, 348-356
- *Journal of Pharmaceutical Sciences,* 2009, vol. 98 (9
- *Diabetes Technology & Therapeutics,* 2009, vol. 11 (6
- *Journal of the Japan Endocrine Society,* 1978, vol. 54 (5), 676-691
- *Tissue Eng.,* 2001, vol. 7 (1), 35-44
- *Diabetologia,* 2000, vol. 43 (10), 1319-1328
- *Igaku no Ayumi,* 1983, vol. 125 (10), 835-843
- *J. Immunol.,* 1992, vol. 148, 1149
- **WALLHAUSER, K.H.** Develop. Biol. Standard. S. Kranger, 1974, vol. 24, 9-28